# EUROPÄISCHE PATENTSCHRIFT

(11) **EP 0 923 521 B1**
(45) Veröffentlichungstag und Bekanntmachung des Hinweises auf die Patenterteilung: **22.08.2001**
(21) Anmeldenummer: 97941903.3
(22) Anmeldetag: 29.07.1997
(51) Int. Cl.: C07C 45/50, C07H 15/12, C07H 15/14, C08B 37/16

(54) **VERFAHREN ZUR HYDROFORMYLIERUNG VON OLEFINEN IM ZWEIPHASENSYSTEM**
OLEFIN HYDROFORMYLATION PROCESS IN A TWO-PHASE SYSTEM
PROCEDE POUR L'HYDROFORMYLATION D'OLEFINES DANS LE SYSTEME A DEUX PHASES

(30) Priorität: 02.08.1996 DE 19631322
(43) Veröffentlichungstag der Anmeldung: 23.06.1999
(73) Patentinhaber: Studiengesellschaft Kohle mbH, 45470 Mülheim an der Ruhr (DE)
(72) Erfinder: REETZ, Manfred, Theodor, D-45470 Mülheim an der Ruhr (DE); WALDVOGEL, Siegfried, D-45470 Mülheim an der Ruhr (DE)
(74) Vertreter: von Kreisler, Alek, Dipl.-Chem.
(86) Internationale Anmeldenummer: EP9704117
(87) Internationale Veröffentlichungsnummer: WO9805618

(56) Entgegenhaltungen:
- WO-A-96/22267
- E.MONFLIER ET AL.: "A Further Breakthrough in Biphasic, Rhodium-Catalyzed Hydroformylation : the Use of Per (2,6-di-O-methyl)-B-Cyclodextrin as Inverse Phase Transfer Catalyst." TETRAHEDRON LETTERS., Bd. 36, Nr. 52, 25.Dezember 1995, OXFORD GB, Seiten 9481-9484, XP002052912 in der Anmeldung erwähnt

## Beschreibung

Die vorliegende Erfindung betrifft ein Verfahren zur Hydroformylierung von terminalen und internen Olefinen in einem Zweiphasensystem unter Verwendung von neuen Metallkatalysatoren. Das Zweiphasensystem besteht aus einer wäßrigen und einer organischen Phase. Die Metalle gehören zur VIII Gruppe des Periodensystems, z. B. Rh, Ru, Ir, Co oder Pd. Die Liganden für die Metalle sind neuartige Phosphan-modifizierte β-Cyclodextrine, die wasserlöslich sind.

Die bei der Hydroformylierung gebildeten Aldehyde sind industriell nützliche Verbindungen, die entweder direkt Anwendung finden, z. B. als Duftstoffe, oder als Zwischenstufe für die Produktion von weiteren Stoffklassen, so z. B. bei der Herstellung von Lösungsmitteln, Waschmitteln, Duftstoffen, Pharmaka oder Weichmachern [K. Weissermel, H. J. Arpe, *Industrial Organic Chemistry*, VCH, Weinheim, 1993].

Kommerziell werden die von Roelen eingeführten Cobalt-Katalysatoren, z. B. Co₂(CO)₈, bei 150 - 180 °C/15 - 20 MPascal (150 - 180 °C/150 - 200 atm) verwendet, Bedingungen, die zu Nebenreaktionen wie Hydrierung, Isomerisierung und Aldehydkondensation führen. Im Rahmen des sogenannten Low Pressure Oxo Process werden in organischen Lösungsmitteln lösliche Rhodium-Komplexe wie HRh(CO)(PPh₃)₃ als Katalysatoren verwendet, wobei man bei 90 - 110 °C/2 MPascal (90 - 110 °C/ 20 atm) arbeitet und somit weniger unerwünschte Nebenprodukte erhält. Dieses Verfahren sowie andere homogenkatalysierte Hydroformylierungen werden hauptsächlich zur Produktion von Butyraldehyd und anderen leicht siedenden Aldehyden angewandt. Jedoch ist bei homologen bzw. höher siedenden Aldehyden die destillative Abtrennung vom Katalysator problematisch [G. W. Parshall, S. D. Itell, *Homogeneous Catalysis*, Wiley, New York, 1992]. Aus diesem Grund gibt es weltweit industrielle Bestrebungen, Hydroformylierungen im Zweiphasensystem durchzuführen [B. Cornils, in *New Synthesis with Carbon Monoxide* (Hrsg. J. Falbe), Springer Verlag, New York, 1980; B. Cornils, E. Wiebus, *Chemtech.* **1995**, 33]. Dazu werden wasserlösliche Phosphane, z. B. P(C₆H₄SO₃Na)₃, als Liganden für das Rhodium verwendet (TPPTS-Rh-System). Der Rh-Katalysator hält sich in der wäßrigen Phase auf, während sich Olefin und Produkt (Aldehyd) in der organischen Phase befinden. Hoechst/Rhone-Poulenc produziert so n-Butyraldehyd aus Propen [B. Cornils, E. Wiebus, *Chem. Ing. Tech.* **66** (1994) 916; W. A. Herrmann, et al, *J. Mol. Catal.* **97** (1995) 65]. Leider ist dieses einfache Verfahren auf Ethylen und Propen beschränkt, die eine zwar kleine, aber doch genügend hohe Wasserlöslichkeit aufweisen, um in der wäßrigen bzw. katalysatorhaltigen Phase umgesetzt zu werden. Homologe Olefine wie z. B. n-Hexen oder n-Octen werden in diesem System praktisch gar nicht oder nur schlecht umgesetzt. Deshalb hat es nicht an Versuchen gefehlt, dieses Problem zu lösen, z. B. mit Hilfe von Tensiden bzw. Phasentransfer-Katalysatoren, speziellen Liganden oder Lösungsmitteln, jedoch nur mit begrenztem Erfolg, wie kürzlich von E. Monflier zusarnmengefaßt [*Angew. Chem.* **107** (1995) 2450; *Tetrahedron Lett.* **36** (1995) 9481]. Eine Verbesserung beschreibt E. Monflier [s. o.], wonach dem Zweiphasensystem β-Cyclodextrinderivate (β-CD-Derivate) zugesetzt werden, die als Lösungsvermittler dienen. Im Falle von längerkettigen Olefinen wie 1-Octen oder 1-Decen steigt dadurch die Aktivität des Katalysatorsystems maximal um etwa das 10fache, so daß brauchbare Ausbeuten an den entsprechenden Aldehyden erhalten werden, und zwar mit n/iso-Verhältnissen von etwa 2:1. Allerdings beträgt die chemische Selektivität in der Regel nur 85 - 90%, d. h. es werden 10 - 15% Nebenprodukte erhalten. Innere Olefine wie z. B. 5-Decen werden praktisch gar nicht hydroformyliert (nur 3% Umsatz), d. h. das Katalysatorsystem ist nicht sehr aktiv. Von großem Nachteil ist auch die Tatsache, daß ein großer Überschuß an β-Cyclodextrinderivat nötig ist; typischerweise beträgt das Verhältnis Rh : P(C₆H₄SO₃Na)₃: β-Cyclodextrinderivat etwa 1 : 8 : 14. Der Vorteil der mäßigen Aktivitätssteigerung durch Verwendung von CD-Derivaten relativiert sich angesichts der genannten Nachteile.

Die vorliegende Erfindung beinhaltet ein Zweiphasensystem (H₂O/organische Phase) mit einem wasserlöslichen Katalysator, der die glätte Hydroformylierung von den unterschiedlichsten terminalen und inneren Olefinen mit hoher Selektivität (>99%) ermöglicht, und zwar unter erstaunlich milden Bedingungen. Dabei handelt es sich um ein überraschend aktives Katalysatorsystem. So ist die Katalysatoraktivität bei der Hydroformylierung von längerkettigen 1-Olefinen wie 1-Octen etwa 150 mal so hoch wie bei dem bekannten TPPTS-Rh-System.

Es ist bekannt, daß 1) β-Cyclodextrin (β-CD) sowie viele β-CD-Derivate wasserlöslich sind, und 2) solche Verbindungen effiziente Wirtmoleküle darstellen, die in ihren inneren hydrophoben Hohlräumen aliphatische und aromatische Kohlenwasserstoffe reversible einfangen bzw. binden können. Enthält nun das β-CD ein metalltragendes Diphosphan, so können die in der organischen Phase vorhandenen und zu hydroformylierenden Olefine vom β-CD eingefangen und spontan katalytisch umgesetzt werden.

Die im Rahmen der vorliegenden Erfindung synthetisierten Phosphanliganden gehören zwei Klassen an: Typ I mit freien Hydroxygruppen und Typ II mit voll oder partiell alkylierten Hydroxygruppen.

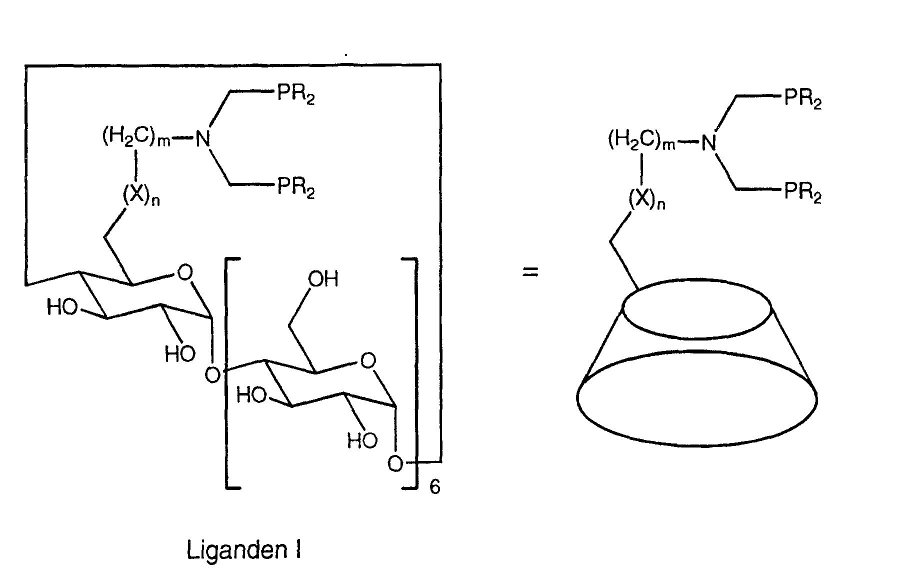

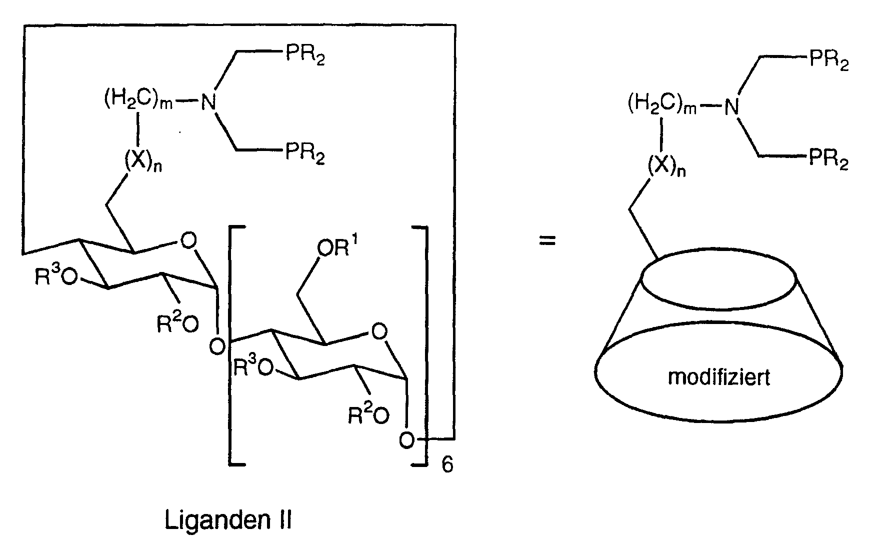

Aus den Formelbildern I/II geht hervor, daß ein chelatisierendes Diphosphan über einen Spacer an β-CD gebunden ist. Im einfachsten Fall ist der Spacer sehr kurz, d. h. n = m = 0. Dieser Ligand ist häufig bevorzugt. Es ist aber auch möglich, daß X ein Heteroatom wie Sauerstoff, Schwefel oder Stickstoff (als NR'-Fragment mit R' = H, Alkyl, Aryl) ist, wobei in diesen Fällen n = 1 ist. Die Länge des restlichen Teils des Spacers kann bequem variiert werden, z. B, m = 0 - 6, vorzugsweise ist m = 2 - 4. Die R-Reste sind aliphatisch wie Ethyl, Isopropyl oder Cyclohexyl, oder vorzugsweise aromatisch wie z. B. Phenyl oder Naphthyl oder substituierte Vertreter wie z. B. o-, m- oder p-Methyl, o-, m-, p-Chlor, o-, m- oder p-Amino, oder vorzugsweise o-, m- oder p-SO₃Na.

Was die Reste R¹, R² und R³ im β-CD-Teil der Liganden II angeht, so stellen sie Alkylreste dar, insbesondere:
R¹ = R² = R³ = CH₃, CH₂=CHCH₂, C₆H₅CH₂, HOCH₂CH(OH)CH₂, CH₃CH(OH)CH₂
oder
R¹ = CH₃, CH₂=CHCH₂, C₆H₅CH₂, HOCH₂CH(OH)CH₂, CH₃CH(OH)CH₂
R² = R³ = H
oder
R¹ = H
R² = R³ = CH₃, CH₂=CHCH₂, C₆H₅CH₂, HOCH₂CH(OH)CH₂, CH₃CH(OH)CH₂
oder
R¹ = R³ = H
R² = CH₃, CH₂=CHCH₂, C₆H₅CH₂, HOCH₂CH(OH)CH₂, CH₃CH(OH)CH₂
oder
R¹ = R² = CH₃, CH₂=CHCH₂, C₆H₅CH₂, HOCH₂CH(OH)CH₂, CH₃CH(OH)CH₂ R³ = H

Zur Bildung der Metallkatalysatoren oder Präkatalysatoren werden die Liganden mit Salzen der Metalle der VIII Gruppe des Periodensystems umgesetzt, insbesondere werden dabei Salze von Rh, Ru, Ir, Co oder Pd verwendet, vorzugsweise Rhodiumsalze. Typischerweise wird Rh(COD)₂BF₄ eingesetzt. Dabei kann man entweder so vorgehen, daß die daraus entstandenen Metallkomplexe isoliert und charakterisiert und dann erst in der Hydroformylierung eingesetzt werden, oder daß eine in situ Reaktionsführung gewählt wird, bei der Ligand und Metallsalz dem Reaktionsgemisch einfach zugesetzt werden.

Die Hydroformylierung wird im Zweiphasensystem durchgeführt, wobei es um eine organische und eine katalysatorhaltige wäßrige Phase geht. Die organische Phase besteht aus einem umzusetzenden Olefin oder aus dem Olefin und einem inerten Lösungsmittel wie Toluol oder Diethylether. Vorzugsweise wird kein organisches Lösungsmittel verwendet, d. h. die organische Phase ist das Olefin selbst. Der wäßrigen Phase wird ggf. kleine Mengen eines Additivs in Form von einem polaren Solvens wie z. B. Dimethylformamid zugegeben.

Zur Durchführung der Hydroformylierung wird auf das Zweiphasensystem im geschlossenen Reaktionsgefäß eine Mischung aus CO und H₂ aufgepreßt, gewöhnlich im Verhältnis von 1:1. Der Druck während der Hydroformylierung kann von 0,1 bis 30 MPascal (1 bis 300 atm) variiert werden, vorzugsweise liegt er im Bereich von 2 bis 10 Mpascal (20 bis 100 atm). Ein Temperaturbereich von 50 °C bis 120 °C kann gewählt werden, vorzugsweise liegt die Temperatur zwischen 60 °C und 80 °C.

Das erfindungsgemäße Zweiphasen-Katalysatorsystem ist bei der Hydroformylierung von 1-Alkenen, internen Alkenen, 1,1-disubstituierten Alkenen und trisubstituierten Alkenen deutlich aktiver als das herkömmliche Zweiphasensystem unter Verwendung von wasserlöslichem P(C₆H₄SO₃Na)₃ und Rh-Salzen (vgL Hoechst/Rhone-Poulenc-TPPTS-Rh-System). Während z. B. 1-Octen mit dem letztgenannten Katalysatorsystem praktisch gar nicht hydroformyliert wird, kann mit Rh-Komplexen der β-CD -modifizierten Liganden unter vergleichbaren Bedingungen (80 °C/18 h/5 MPascal (50 atm)) ein vollständiger Umsatz mit >99% Selektivität erreicht werden. Die Steigerung der Katalysatoraktivität beim Übergang vom TPPTS-Rh-Zweiphasensystem zum erfindungsgemäßen Zweiphasenkatalysatorsystem beträgt einen Faktor von mehr als 150. Überraschenderweise gehen mit dem erfindungsgemäßen Zweiphasen-Katalysatorsystem auch notorisch unreaktive Olefine wie cis- oder trans-3-Hexen und sogar trisubstituierte Alkene wie 1-Methylcyclopenten glatte Hydroformylierungen ein.

Zur Synthese der Liganden wird zunächst β-Cyclodextrin selektiv in das in der Literatur bekannte Mono(6-O-p-toluolsulfonyl)-ß-Cyclodextrin überführt. Die O-Tosyl-Gruppen werden im Zuge einer nucleophilen Substitution durch ein Heteroatom verdrängt. Nach Erhalt der entsprechenden primären Amine werden diese in bekannter Weise mit (HOCH₂)₂P⁺R₂X⁻ unter Bildung der gewünschten β-CD-modifizierten Diphosphanliganden vom Typ I und II umgesetzt.

Neben der einfachen Darstellung der Liganden und der hohen Katalysatoraktivität und Selektivität stellt die einfache Rückgewinnung des Katalysators in Form der wäßrigen Phase einen weiteren Vorteil dar.

### Beispiel 1: 6^{A}-N,N-Bis(diphenylphosphinomethyl)amino-6^{A}-desoxy-β-cyclodextrin

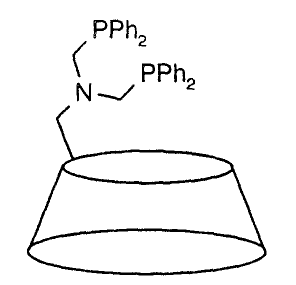

6-Desoxy-6-p-toluolsulfonyl-β-cyclodextrin: Durch direkte Tosylierung in Wasser (500 g Cyclodextrin in 3200 ml Wasser) mit Natriumhydroxid als Base und p-Tosylchlorid erhalten. Die Reaktion wird in offenen Behältern bei Raumtemperatur durchgeführt. Zur Einbringung des Tosylchlorids werden kleine Mengen an Aceton verwendet. Fällung erfolgt in der Kälte (0°C), Umkristallisation aus Wasser ergibt das Produkt in 8% Ausbeute mit einer HPLC-Reinheit >94%.
Bei der Verwendung von weniger Wasser (2500 ml) können nach Umkristallisation auf 28 % Produkt erhalten werden. Das so erhaltene Produktgemisch enthält ca. 85 % 6-Desoxy-6-p-toluolsulfonyl-β-cyclodextrin Rest ist unfunktionallisiertes β-Cyclodextrin.

6^{A}-Azido-6^{A}-desoxy-β-cyclodextrin: Durch nucleophile Substitution von 6-Desoxy-6-p-toluolsulfonyl-β-Cyclodextrin mit *N,N,N',N'*-Tetramethylguanidiniumazid in DMF erhalten (90°C, 48 h). Fällung erfolgt mit Aceton. Eine Umkristallisation in Ethanol/Wasser ergibt farblose Kristalle mit einer von Ausbeute von 92 % und einer HPLC-Reinheit >93%.
IR:ν/cm⁻¹(KBr): 2105 (ss, N₃)

6^{A}-Amino-6^{A}-desoxy-β-cyclodextrin: Durch katalytische Reduktion von 6^{A}-Azido-6^{A}-desoxy-β-cyclodextrin mit Platindioxid (0.5 %) und molekularem Wasserstoff (0,1 MPascal (1 atm)). Als Lösungsmittel dient Wasser (25 °C, 8h). Rekationskontrolle erfolgt via IR. Nach Aufarbeitung resultiert ein leicht gräuliches Pulver mit einer Ausbeute von 79 %.

6^{A}-*N,N*-Bis(diphenylphosphinomethyl)amino-6^{A}-desoxy-β-cyclodextrin: 1.5 g (5.3 mmol) Bis(hydroxymethyl)diphenylphosphoniumchlorid und 2.8 g (2.4 mmol) 6^{A}-Amino-6^{A}-desoxy-β-cyclodextrin werden in einem Gemisch aus 15 ml Wasser und 30 ml Methanol gelöst. Nach der Zugabe von 1.3 ml (9.4 mmol) Triethylamin trübt sich das Gemisch. Man refluxiert zwei Stunden. Die abgekühlte Reaktionsmischung wird mit 40 ml Wasser versetzt und 18 Stunden bei 0°C aufbewahrt. Die Suspension wird auf eine Schutzgasfritte gehebert. Der Niederschlag wird zweimal mit 10 ml Wasser danach mit 20 ml Aceton gewaschen. Der Rückstand wird am Hochvakuum getrocknet. Zur Reinigung wird der Rückstand Methanol/Benzol umkristallisiert. Man dekantiert die überstehende Lösung ab und trocknet den Feststoff bei 50°C im Diffusionspumpenvakuum. Ausbeute: 3.35 g (2.1 mmol) 91 %.^{**31**}**P-NMR** (80 MHz, d₆-DMSO) : δ = -27.1 ppm. **MS** (FAB/pos. Matrix: Glycerin): m/z = 1530 [M+H]⁺, 1344 [M-PPh₂]⁺, 1158 [M-2PPh₂]⁺. (FAB/neg. Matrix: Glycerin): m/z = 1528 [M-H]⁻, 40 %. (detaillierte NMR-Untersuchung siehe Beispiel zwei).

| **EA**: C₆₈H₉₃N₁P₂O₃₄•0.5 C₆H₆:MG(1568.521) | | | | |
|---|---|---|---|---|
| ber. | C 54.32 | H 6.17 | N 0.89 | P 3.95 |
| gef. | C 54.16 | H 6.07 | N 0.73 | P 3.83 |

### Beispiel 2: S-(2-N,N-Bis(diphenylphosphinomethyl)aminoethyl)-6^{A}-desoxy-6^{A}-thio-β- cyclodextrin

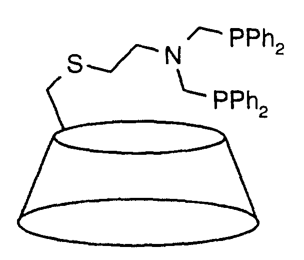

S-(2-Aminoethyl)-6^{A}-desoxy-6^{A}-thio-β-cyclodextrin: Durch nucleophile Substitution von 6-Desoxy-6-p-toluolsulfonyl-β-cyclodextrin mit *N*-(2-Mercapto)ethylphthalimid sowie Cäsiumcarbonat als Base wird die phthalimidogeschützte Verbindung erhalten. Als Solvens dient DMF (80°C, 24 h). Hydrazinolyse und Fällung mit absolutem Ethanol ergeben ein Rohprodukt, das durch Umkristallisation in Methanol/Wasser gereinigt wird. Ausbeute: 76 % (bezogen auf eingesetztes Cyclodextrintosylat).

S-(2-*N*,*N*-Bis(diphenylphosphinomethyl)aminoethyl)-6^{A}-desoxy-6^{A}-thio-β-cyclodextrin: 7.1 g (25 mmol) Bis(hydroxymethyl)diphenylphosphoniumchlorid und 10 g (8.3 mmol) S-(2-Aminoethyl)-6^{A}-desoxy-6^{A}-thio-β-cyclodextrin werden in einem Gemisch aus 60 ml Wasser, 10 ml Benzol und 150 ml Methanol gelöst. Nach der Zugabe von 3.4 ml (24.5 mmol) Triethylamin trübt sich das Gemisch. Man refluxiert zwei Stunden. Beim Abkühlen fällt ein weißer Feststoff aus. Die abgekühlte Reaktionsmischung wird dekantiert und der Rückstand im Hochvakuum bei 50°C getrocknet. Zur Reinigung wird der Rückstand in Methanol/Benzol umkristallisiert. Der polykristalline Festkörper wird isoliert und bei 50°C im Diffusionspumpenvakuum getrocknet. Ausbeute: 9.72 g (6.15 mmol) 73 % (bezogen auf eingesetztes Cyclodextrintosylat).^{**31**}**P-NMR** (100 MHz, d6-DMSO) : δ = -27.40 ppm. **MS** (ESI/pos. in MeOH, H₂O): m/z = 1590 ([M+H]⁺, 100 %), 1392 ([M+H-CH₂PPh₂]⁺, 60 %).

| **EA** C₇₀H₉₇N₁O₃₄P₂S₁•0.5 C₆H₆ MG(1628.532) | | | | |
|---|---|---|---|---|
| ber. | C 53.79 | H 6.19 | N 0.86 | P 3.80 |
| gef. | C 53.69 | H 6.04 | N 1.05 | P 3.63 |

Detaillierte NMR-Untersuchung beispielhaft für alle Derivate aus Beispiel 1-9. Untersuchungen wurden mit einem Bruker DMX-600 gemacht.

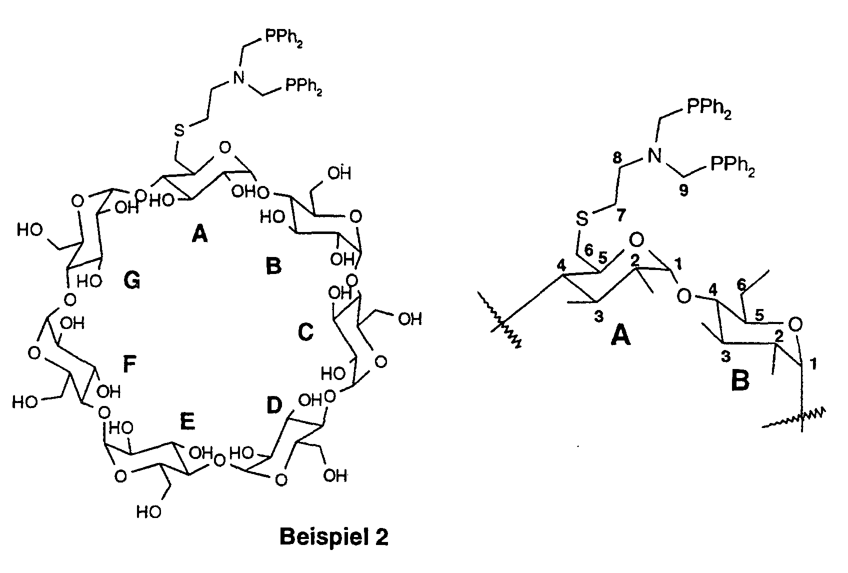

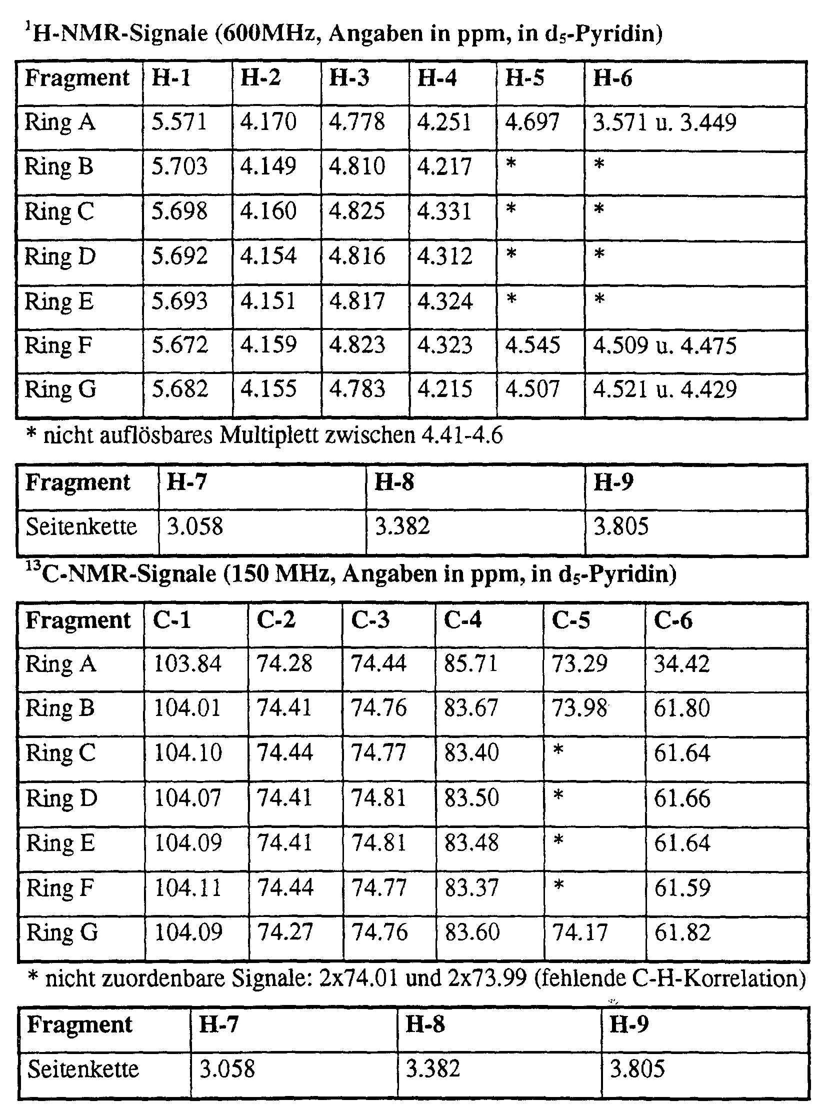

### Beispiel 3: S-(3-N,N-Bis(diphenylphosphinomethyl)aminopropyl)-6^{A}-desoxy-6^{A}-thio-β-cyclodextrin

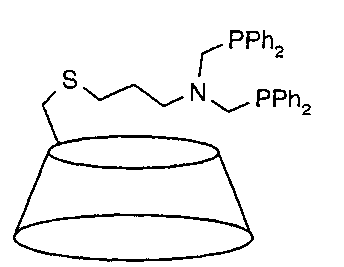

S-(3-Aminopropyl)-6^{A}-desoxy-6^{A}-thio-β-cyclodextrin: Durch nucleophile Substitution von 6-Desoxy-6-p-toluolsulfonyl-β-cyclodextrin mit *N*-(3-Mercapto)propylphthalimid sowie Cäsiumcarbonat als Base wird die phthalimidogeschützte Verbindung erhalten. Als Solvens dient DMF (80°C, 24 h). Hydrazinolyse und Fällung mit absolutem Ethanol ergeben ein Rohprodukt, das durch Umkristallisation in Methanol/Wasser gereinigt wird. Ausbeute: 89 % (bezogen auf eingesetztes Cyclodextrintosylat).

S-(3-*N,N*-Bis(diphenylphosphinomethyl)aminopropyl)-6^{A}-desoxy-6^{A}-thio-β-cyclodextrin: 2.46 g (8.7 mmol) Bis(hydroxymethyl)diphenylphosphoniumchlorid und 3.5 g (2.9 mmol) S-(3-Aminopropyl)-6^{A}-desoxy-6^{A}-thio-β-cyclodextrin werden in einem Gemisch aus 15 ml Wasser und 30 ml Methanol gelöst. Nach der Zugabe von 1.1 ml (7.9 mmol) Triethylamin trübt sich das Gemisch. Man refluxiert zwei Stunden. Beim Abkühlen fällt ein weißer Feststoff aus. Aufarbeitung und Reinigung analog zu Beispiel 2. Ausbeute: 3.92 g (2.5 mmol) 86 %. ^{**31**}**P-NMR** (100 MHz, d₆-DMSO) : δ = -27.20 ppm. **MS** (ESI/pos. in MeOH, H₂O): m/z = 1604 [M+H]⁺.

| **EA** C₇₁H₉₉N₁O₃₄P₂S₁•3 H₂O MG(1657.52) | | | | |
|---|---|---|---|---|
| ber. | C 51.42 | H 6.38 | N 0.84 | P 3.73 |
| gef. | C 51.40 | H 6.54 | N 0.67 | P 3.75 |

### Beispiel 4: S-(4-N,N-Bis(diphenylphosphinomethyl)aminobutyl)-6^{A}-desoxy-6^{A}-thio-β- cyclodextrin

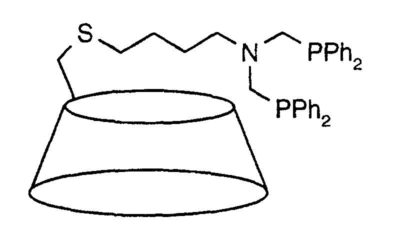

S-(4-Aminobutyl)-6^{A}-desoxy-6^{A}-thio-β-cyclodextrin: Durch nucleophile Substitution von 6-Desoxy-6-p-toluolsulfonyl-β-cyclodextrin mit *N*-(4-Mercapto)butylphthalimid sowie Cäsiumcarbonat als Base wird die phthalimidogeschützte Verbindung erhalten. Als Solvens dient DMF (80°C, 24 h). Hydrazinolyse und Fällung mit absolutem Ethanol ergeben ein Rohprodukt, das durch Umkristallisation in Methanol/Wasser gereinigt wird. Ausbeute: 77 % (bezogen auf eingesetztes Cyclodextrintosylat).

S-(4-*N*,*N*-Bis(diphenylphosphinomethyl)aminobutyl)-6^{A}-desoxy-6^{A}-thio-β-cyclodextrin: 1.0 g (3.5 mmol) Bis(hydroxymethyl)diphenylphosphoniumchlorid und 2.0 g (1.23 mmol) S-(4-Aminobutyl)-6^{A}-desoxy-6^{A}-thio-β-cyclodextrin werden in einem Gemisch aus 10 ml Wasser und 30 ml Methanol gelöst. Nach der Zugabe von 0.9 ml (6.4 mmol) Triethylamin trübt sich das Gemisch. Man refluxiert zwei Stunden. Beim Abkühlen fällt ein weißer Feststoff aus. Aufarbeitung und Reinigung analog zu Beispiel 2. Ausbeute: 1.34 g (0.83 mmol) 67 %. ^{**31**}**P-NMR** (100 MHz, d₆-DMSO) : δ = -27.37 ppm. **MS** (ESI/pos. in MeOH, H₂O): m/z = 1618 [M+H]⁺, 100 %.

| **EA** C₇₂H₁₀₁N₁O₃₄P₂S₁•2 H₂O MG(1653.56) | | | | |
|---|---|---|---|---|
| ber. | C 52.25 | H 6.40 | N 0.85 | P 3.75 |
| gef. | C 52.25 | H 6.13 | N 0.84 | P 3.31 |

### Beispiel 5: 6^{A}-N,N-Bis(diphenylphosphinomethyl)amino-6^{A}-desoxy-per(O-methyl)-β- cyclodextrin

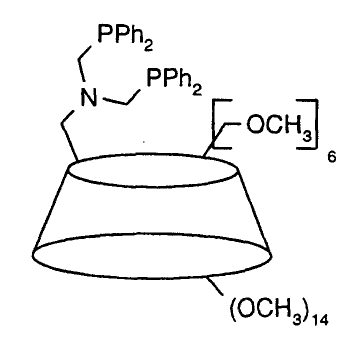

6^{A}-Azido-6^{A}-desoxy-per(O-methyl)-β-cyclodextrin: Durch Methylierung von 6^{A}-Azido-6^{A}-desoxy-β-cyclodextrin mit Methyliodid in DMSO und wasserfreiem Natriumhydroxid als Base. Ausbeute: 94 %. Das Produkt zeigt im HPLC eine Reinheit von >92%. **IR**:ν/cm⁻¹(KBr): 2101 (ss, N₃)

6^{A}-Amino-6^{A}-desoxy-per(O-methyl)-β-cyclodextrin: Durch Reduktion von 6^{A}-Azido-6^{A}-desoxy-per(O-methyl)-β-cyclodextrin mit Natriumborhydrid in siedendem Isopropanol. Reaktionskontrolle erfolgt via IR. Ausbeute: 96 % Das Produkt zeigt im HPLC eine Reinheit von >92%.

6^{A}-*N,N*-Bis(diphenylphosphinomethyl)amino-6^{A}-desoxy-per(O-methyl)-β-cyclodextrin: 1.7 g (6.0 mmol) Bis(hydroxymethyl)diphenylphosphoniumchlorid und 4.0 g (2.8 mmol) 6^{A}-Amino-6^{A}-desoxy-per(O-methyl)-β-cyclodextrin werden in einem Gemisch aus 40 ml Wasser und 40 ml Methanol gelöst. Nach der Zugabe von 1.2 ml (8.6 mmol) Triethylamin trübt sich das Gemisch. Man refluxiert zwei Stunden. Dabei bildet sich eine zweite Phase, die sich absetzt. Die abgekühlte Reaktionsmischung wird dreimal mit 20 ml Dichlormethan extrahiert und mit wenig Magnesiumsulfat getrocknet. Das Lösungsmittel wird im Vakuum abgezogen. Der Rückstand wird in siedendem Hexan aufgenommen und filtriert. Das Produkt fällt bei 0°C als Öl wieder aus. Durch Trocknen am Diffusionspumpenvakuum bei 80°C resultiert ein harter, farbloser Schaum. Die so erhaltene Verbindung ist analysenrein. Ausbeute: 4.79 g (2.6 mmol) 93 %. ^{**1**}**H-NMR** (300 MHz, CDCl₃): δ = 3.05 ppm (dd, ³J = 3.3 Hz, ³J = 9.6 Hz, 7H, H-2), 3.20-3.69 ppm (m, ∼97H, H-3, H-4, H-5, H-6, OCH₃, NH₂), 4.93-5.01 ppm (m, 6H, H-1^{B,C,D,E,F,G}), 5.04 ppm (d, 1H, ³J = 3.5 Hz, H-1^{A}). ^{**31**}**P-NMR** (100 MHz, CD₂Cl₂): δ = -29.00 ppm. **MS** (ESI/pos. in CH₃OH, H₂O): m/z = 1848 ([M+K]⁺, 28 %), 1832 ([M+Na]⁺, 100 %), 1810 ([M+H]⁺, 30 %).

| **EA** C₈₈H₁₃₃N₁P₂O₃₄ MG(1809.818) | | | | |
|---|---|---|---|---|
| ber. | C 58.35 | H 7.41 | N 0.77 | P 3.42 |
| gef. | C 58.32 | H 7.33 | N 0.75 | P 3.47 |

### Beispiel 6: 6^{A}-N,N-Bis(diphenylphosphinomethyl)amino-hepta(O-2,O-3-dimethyl)-6^{A}-desoxy-β-cyclodextrin

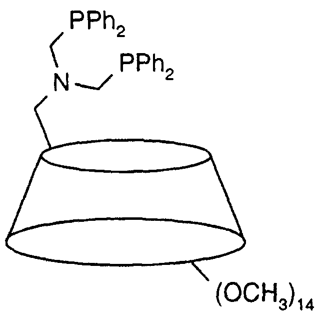

6^{A}-Azido-hexakis(O-6-*tert*-butyldimethylsilyl)-6^{A}-desoxy-β-cyclodextrin: Durch Umsetzung von 6^{A}-Azido-6^{A}-desoxy-β-cyclodextrin mit *tert*-Butyldimethylchlorsilan in Pyridin erhalten. Aufreinigung erfolgt über Säulenchromatographie (Kieselgel, Ethanol/Essigester/Wasser). Ausbeute: 76%.

6^{A}-Azido-hexakis(O-6-*tert*-butyldimethylsilyl)-heptakis(O-2,O-3-dimethyl)-6^{A}-desoxy-β-cyclo-dextrin: Wird durch Methylierung von 6^{A}-Azido-hexakis(O-6-*tert*-butyldimethylsilyl)-6^{A}-desoxy-β-cyclodextrin mit Natriumhydrid und Methyliodid in THF erhalten. Reinigung erfolgt wiederum via Säulenchromatographie (Kieselgel, Methylenchlorid/Methanol) Ausbeute: 95%.

6^{A}-Azido-heptakis(O-2,O-3-dimethyl)-6^{A}-desoxy-β-cyclodextrin: Desilylierung wird durch Refluxieren von 6^{A}-Azido-hexakis(O-6-*tert*-butyldimethylsilyl)-heptakis(O-2,O-3-dimethyl)-6^{A}-desoxy-β-cyclodextrin mit Pyridiniumtosylat in absolutem Ethanol erreicht. Zur Aufarbeitung wird ein Mixed-Bed-Ionenaustauscherverwendet. Ausbeute: 96%.

6^{A}-Amino-heptakis(O-2,O-3-dimethyl)-6^{A}-desoxy-β-cyclodextrin: Durch katalytische Reduktion von 6^{A}-Azido-heptakis(O-2,O-3-dimethyl)-6^{A}-desoxy-β-cyclodextrin mit Platindioxid und molekularem Wasserstoff (1 atm). Reaktionskontrolle erfolgt via IR. Nach Aufarbeitung resultiert ein leicht gelblicher Schaum mit einer Ausbeute von 75 %.

6^{A}-*N,N*-Bis(diphenylphosphinomethyl)amino-heptakis(O-2,O-3-dimethyl)-6^{A}-desoxy-β-cyclo-dextrin: 1.0 g (3.3 mmol) Bis(hydroxymethyl)diphenylphosphoniumchlorid und 1.8 g (1.35 mmol) 6^{A}-Amino-6^{A}-desoxy-hepta(O-2,O-3-dimethyl)-β-cyclodextrin werden in einem Gemisch aus 12 ml Wasser und 12 ml Methanol gelöst. Nach der Zugabe von 0.7 ml (5.0 mmol) Triethylamin trübt sich das Gemisch. Man refluxiert zwei Stunden. Dabei bildet sich eine weiße Emulsion. Die abgekühlte Reaktionsmischung wird dreimal mit 20 ml Dichlormethan extrahiert und mit wenig Magnesiumsulfat getrocknet. Das Lösungsmittel wird im Vakuum abgezogen. Der Rückstand wird in Hexan/Diethylether = 1 : 1 aufgenommen und filtriert. Das Produkt fällt bei 0°C als Öl wieder aus. Durch Trocknen am Diffusionspumpenvakuum bei 80°C resultiert ein harter, gelblicher Schaum. Die so erhaltene Verbindung ist analysenrein. Ausbeute: 1.73 g (1.0 mmol) 74 %. ^{**31**}**P-NMR** (100 MHz, CD₂Cl₂) : δ = -28.70 ppm. **MS** (ESI/pos. in CH₃OH, H₂O): m/z = 1765 ([M+K]⁺, 5 %), 1749 ([M+Na]⁺, 100 %), 1727 ([M+H]⁺, 55 %).

| **EA** C₈₂H₁₂₁N₁P₂O₃₄ MG(1725.72) | | | | |
|---|---|---|---|---|
| ber. | C 57.02 | H 7.07 | N 0.81 | P 3.59 |
| gef. | C 56.71 | H 7.33 | N 0.73 | P 3.41 |

### Beispiel 7: 6^{A}-N,N-Bis(diphenylphosphinomethyl)amino-hepta(O-methyl)-6^{A}-desoxy-β-cyclodextrin

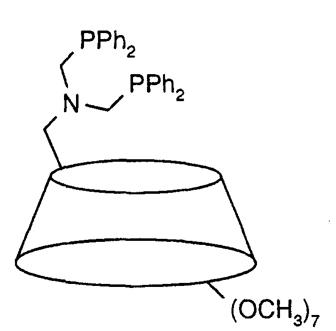

6^{A}-Azido-hexakis(O-6-*tert*-butyldimethylsilyl)-hepta(O-methyl)-6^{A}-desoxy-β-cyclodextrin: Wird durch vorsichtige Methylierung von 6^{A}-Azido-hexakis(O-6-*tert*-butyldimethylsilyl)-6^{A}-desoxy-β-cyclodextrin mit Natriumhydrid und Methyliodid in THF erhalten. Reinigung erfolgt wiederum via Säulenchromatographie (Kieselgel, Methylenchlorid/Methanol) Ausbeute: 83 %.

6^{A}-Azido-hepta(O-methyl)-6^{A}-desoxy-β-cyclodextrin: Desilylierung wird durch Refluxieren von 6^{A}-Azido-hexakis(O-6-*tert*-butyldimethylsilyl)-hepta(O-methyl)-6^{A}-desoxy-β-cyclodex-trin mit Pyridiniumtosylat in absolutem Ethanol erreicht. Zur Aufarbeitung wird ein Mixed-Bed-Ionenaustauscherverwendet. Ausbeute: 77 %.

6^{A}-Amino-hepta(O-methyl)-6^{A}-desoxy-β-cyclodextrin: Durch katalytische Reduktion von 6^{A}-Azido-hepta(O-methyl)-6^{A}-desoxy-β-cyclodextrin mit Platindioxid und molekularem Wasserstoff (0,1 MPascal (1 atm)). Reaktionskontrolle erfolgt via IR. Nach Aufarbeitung resultiert ein leicht gelblicher Schaum mit einer Ausbeute von 98 %.

6^{A}-*N*,*N*-Bis(diphenylphosphinomethyl)amino-hepta(O-methyl)-6^{A}-desoxy-β-cyclodextrin: 1.2 g (4.2 mmol) Bis(hydroxymethyl)diphenylphosphoniumchlorid und 2.1 g (1.7 mmol) 6^{A}-Amino-6^{A}-desoxy-hepta(O-dimethyl)-β-cyclodextrin werden in einem Gemisch aus 15 ml Wasser und 15 ml Methanol gelöst. Nach der Zugabe von 0.8 ml (5.8 mmol) Triethylamin trübt sich das Gemisch. Man refluxiert zwei Stunden. Dabei bildet sich eine weiße Emulsion. Die abgekühlte Reaktionsmischung wird dreimal mit 20 ml Dichlormethan extrahiert und mit wenig Magnesiumsulfat getrocknet. Das Lösungsmittel wird im Vakuum abgezogen. Der Rückstand wird in Hexan/Diethylether = 1 : 4 aufgenommen und filtriert. Das Produkt fällt bei 0°C als Öl wieder aus. Durch Trocknen am Diffusionspumpenvakuum bei 80°C resultiert ein harter, gelblicher Schaum. Die so erhaltene Verbindung ist analysenrein. Ausbeute: 1.2 g (0.74 mmol) 44 %. ^{**31**}**P-NMR** (100 MHz,[D₆]DMSO: δ = -28.70 ppm. **MS** (ESI/pos. in CH₃OH, H₂O): m/z = 1668([M+K]⁺, 5 %),1651 ([M+Na]⁺, 17 %),1629 ([M+H)⁺,25%).

### Beispiel 8: [S-(2-N,N-Bis(diphenylphosphinomethyl)aminoethyl)-6^{A}-desoxy-6^{A}-thio-β-cyclodextrin]-[η²,η²-Cycloocta-1,5-dien]-rhodium(I)- tetrafluoroborat

Unter Argonatmosphäre werden 1.0 g (0.62 mmol)S-(2-*N,N*-Bis(diphenylphosphinomethyl)-aminoethyl)-6^{A}-desoxy-6^{A}-thio-β-cyclodextrin (Ligand aus Beispiel 2) und 251 mg (0.62 mmol) Bis(η²,η²-Cycloocta-1,5-dien)-rhodium(I)-tetrafluoroborat werden in einem Gemisch aus 30 ml Methanol und 10 ml Dichlormethan suspendiert und 24 h bei Raumtemperatur gerührt. Alle flüchtigen Bestanteile werden im Vakuum entfernt. Der Rückstand wird mehrmals mit heißem Dichlormethan extrahiert. Beim Trocknen am Hochvakuum fällt ein oranges Pulver an. Ausbeute 1.08 g (0.57 mmol) 92 %. ^{**31**}**P-NMR** (600 MHz, d₆-DMSO) : δ = +8.05 ppm, ¹J_{RhP} = 143 Hz. **MS** (ESI/pos. in CH₃OH, H₂O): m/z = 1818 ([M-Cl+H₂O]⁺, 20 %), 1800 ([M-Cl]⁺, 100 %).

| **EA** C₇₈H₁₀₉N₁P₂O₃₄S₁B₁F₄Rh₁ MG(1887.51) | | | | |
|---|---|---|---|---|
| ber. | C 49.49 | H 5.82 | Rh 5.45 | P 3.28 |
| gef. | C 49.23 | H 5.71 | Rh 4.75 | P 3.66 |

### Beispiel 9:: [S-(2-N,N-Bis(diphenylphosphinomethyl)aminoethyl)-6^{A}-desoxy-6^{A}-thio-β- cyclodextrin]-chloro-(η⁶-cymol)-ruthenium(II)chlorid

Unter Argonatmosphäre werden 1.0 g (0.62 mmol)S-(2-*N,N*-Bis(diphenylphosphinomethyl)-aminoethyl)-6^{A}-desoxy-6^{A}-thio-β-cyclodextrin (Ligand aus Beispiel 2) und 215 mg (0.35 mmol) Di-µ-chloro-dichloro-bis[(p-cymol)-ruthenium(II)] werden in einem Gemisch aus 30 ml Methanol und 10 ml Dichlormethan suspendiert und 24 h bei Raumtemperatur gerührt. Alle flüchtigen Bestanteile werden im Vakuum entfernt. Der Rückstand wird mehrmals mit heißem Dichlormethan extrahiert. Beim Trocknen am Hochvakuum fällt ein hellrotes Pulver an. Ausbeute 1.12 g (0.59 mmol) 95 %. ^{**31**}**P-NMR** (100 MHz, d₆-DMSO) : δ = +21.21 ppm. **MS** (FAB/pos., Matrix:Glycerin): m/z = 1861 ([M-Cl]⁺, 100 %).

| **EA** C₈₀H₁₁₁N₁P₂O₃₄S₁Ru₁Cl₂ MG(1895.46) | | | | |
|---|---|---|---|---|
| ber. | C 50.65 | H 5.90 | Ru 5.90 | P 3.27 |
| gef. | C 48.99 | H 5.88 | Ru 6.23 | P 3.11 |

### Beispiel 10: [S-(2-N,N-Bis(diphenylphosphinomethyl)aminoethyl)-6^{A}-desoxy-6^{A}-thio-β- cyclodextrin]-palladiumdichlorid

Unter Argonatmosphäre werden 1.0 g (0.62 mmol)S-(2-*N,N*-Bis(diphenylphosphinomethyl)-aminoethyl)-6^{A}-desoxy-6^{A}-thio-β-cyclodextrin (Ligand aus Beispiel 2) und 250 mg (0.87 mmol) (1,5-Cyclooctadien)-Palladiumdichlorid in 20 ml Methanol suspendiert und 24 h bei Raumtemperatur gerührt. Der Rückstand wird isoliert und fünf mal 10 ml Methanol gewaschen. Nach Trocknung am Hochvakuum bei 50°C erhält man ein gelbes, luftstabiles Pulver. Ausbeute: 878 mg (0.5 mmol) 80 %. ^{**31**}**P-NMR** (100 MHz, d₆-DMSO) : δ = +8.1 ppm. **MS** (ESI/pos. in CH₃OH, H₂O): m/z = 1788 ([M+Na]⁺, 60 %), 1730 ([M-Cl]⁺, 100 %).

| **EA** C₇₀H₉₇N₁P₂O₃₄S₁Pd₁Cl₂ MG(1766.32) | | | | |
|---|---|---|---|---|
| ber. | C 47.58 | H 5.54 | Pd 6.00 | P 3.51 |
| gef. | C 47.31 | H 5.72 | Pd 6.17 | P 3.49 |

### Beispiel 11: Hydroformylierung von 1-Octen mit Ligand aus Beispiel 1

In einem 50 ml Schlenk werden bei Raumtemperatur 2.4x10⁻⁵ mol Ligand (hier: 6^{A}-*N,N*-Bis(diphenylphosphinomethyl)amino-6^{A}-desoxy-β-cyclodextrin) eingewogen und mit 5 ml DMF gelöst. Man versetzt mit 2x10⁻⁵ mol Rh(cod)₂BF₄ (1 ml, Stammlösung 0.02 M in DMF) und rührt 10 min. nach. Es wird mit Wasser auf ein Gesamtvolumen von 20 ml aufgefüllt. Man versetzt mit 10 ml 1-Octen. Der so bereitete Katalyseansatz wird mit einem Heberschlauch im Argon-Gegenstrom in den zuvor bei 80°C im Trockenschrank ausgeheizten und mit Argon begasten Autoklaven mit Tefloneinsatz und Rührkern überführt. Der Autoklav wird verschlossen und mit 10 MPascal (100 bar )Synthesegas versetzt. Unter maximaler Rührung wird auf die gewünschte Reaktionstemperatur aufgeheizt und der Autoklav während der Reaktion auf 60°C gehalten. Nach 18 h wird die Reaktion abgebrochen, den Überdruck an Synthesegas abgelassen und die Phasen im Scheidetrichter getrennt. Die org. Phase wird über wenig Magnesiumsulfat getrocknet und filtriert.
Der Umsatz und die Selektivitäten zu den Produkten werden im ¹H-NMR (200 MHz, CDCl₃) bestimmt. Als diagnostisches Merkmal werden die aldehydischen Signale verwendet. Mit Hilfe der GC/MS wird die Struktur der Produkte bestätigt. Isomerisierungs- und Hydrierungsprodukte werden via Gaschromatographie identifiziert.
Umsatz: 95 % (TON = 3011), Aldehydselektivität: >99 %, n/iso = 3.38

### Beispiel 12:

Hydroformylierung von 1-Octen mit gleicher Vorgehensweise, wie in Beispiel 10 beschrieben, jedoch mit Ligand aus Beispiel 2 (S-(2-*N*,*N*-Bis(diphenylphosphinomethyl)aminoethyl)-6^{A}-desoxy-6^{A}-thio-β-cyclodextrin).
Umsatz: 100 % (TON = 3170), Aldehydselektivität: >99 %, n/iso = 3.0

### Beispiel 13:

Hydroformylierung von 1-Octen mit gleicher Vorgehensweise, wie in Beispiel 11 beschrieben, jedoch nur mit Wasser und ohne DMF als Cosolvens.
Umsatz: 95 % (TON = 3011), Aldehydselektivität: >99 %, nliso = 3.0

### Beispiel 14:

Hydroformylierung von 1-Octen mit gleicher Vorgehensweise, wie in Beispiel 10 beschrieben, jedoch mit Ligand aus Beispiel 3 (S-(3-*N,N*-Bis(diphenylphosphinomethyl)-aminopropyl)-6^{A}-desoxy-6^{A}-thio-β-cyclodextrin). Umsatz: 100 % (TON = 3170), Aldehydselektivität: >99 %, n/iso = 2.25

### Beispiel 15:

Hydroformylierung von 1-Octen mit gleicher Vorgehensweise, wie in Beispiel 10 beschrieben, jedoch mit Ligand aus Beispiel 4 (S-(4-*N,N*-Bis(diphenylphosphinomethyl)-aminobutyl)-6^{A}-desoxy-6^{A}-thio-β-cyclodextrin). Umsatz: 100 % (TON = 3170), Aldehydselektivität: >99 %, n/iso = 2.0

### Beispiel 16:

Hydroformylierung von 1-Octen mit gleicher Vorgehensweise, wie in Beispiel 10 beschrieben, jedoch mit Ligand aus Beispiel 5 (6^{A}-*N,N*-Bis(diphenylphosphinomethyl)amino-6^{A}-desoxy-per(O-methyl)-β-cyclodextrin). Umsatz: 100 % (TON = 3170), Aldehydselektivität: >99 %, n/iso = 1.91

### Beispiel 17:

Hydroformylierung von 1-Octen mit gleicher Vorgehensweise, wie in Beispiel 10 beschrieben, jedoch mit Ligand aus Beispiel 6 (6^{A}-*N,N*-Bis(diphenylphosphinomethyl)amino-heptakis(O-2,O-3-dimethyl)-6^{A}-desoxy-β-cyclo-dextrin).
Umsatz: 100 % (TON = 3170), Aldehydselektivität: >99 %, n/iso = 2.7

### Beispiel 18:

Hydroformylierung von 1-Octen mit gleicher Vorgehensweise, wie in Beispiel 10 beschrieben, jedoch mit Ligand aus Beispiel 7 (6^{A}-*N,N*-Bis(diphenylphosphinomethyl)amino-hepta(O-methyl)-6^{A}-desoxy-β-cyclodextrin).
Umsatz: 100 % (TON = 3170), Aldehydselektivität: >99 %, n/iso = 3.0

### Beispiel 19:

Hydroformylierung von 10 ml 1-Hexen mit gleicher Vorgehensweise, wie in Beispiel 10 beschrieben, jedoch mit Ligand aus Beispiel 2 (S-(2-*N,N*-Bis(diphenylphosphinomethyl)aminoethyl)-6^{A}-desoxy-6^{A}-thio-β-cyclodextrin).
Umsatz: 81 % (TON = 3246), Aldehydselektivität: >99 %, n/iso = 3.1

### Beispiel 20:

Hydroformylierung von 10 ml 1-Decen mit gleicher Vorgehensweise, wie in Beispiel 10 beschrieben, jedoch mit Ligand aus Beispiel 2 (S-(2-*N,N*-Bis(diphenylphosphino-methyl)aminoethyl)-6^{A}-desoxy-6^{A}-thio-β-cyclodextrin).
Umsatz: 89 % (TON = 2342), Aldehydselektivität: >99 %, n/iso = 3.2

### Beispiel 21:

Hydroformylierung von 10 ml 1-Dodecen mit gleicher Vorgehensweise, wie in Beispiel 10 beschrieben, jedoch mit Ligand aus Beispiel 2 (S-(2-*N,N*-Bis(diphenylphosphinomethyl)aminoethyl)-6^{A}-desoxy-6^{A}-thio-β-cyclodextrin).
Umsatz: 94 % (TON = 2139), Aldehydselektivität: >99 %, n/iso = 3.4

### Beispiel 22:

Hydroformylierung von 10 ml Vinylcyclohexan mit gleicher Vorgehensweise, wie in Beispiel 10 beschrieben, jedoch mit Ligand aus Beispiel 2 (S-(2-*N,N*-Bis(diphenylphosphinomethyl)aminoethyl)-6^{A}-desoxy-6^{A}-thio-β-cyclodextrin).
Reaktionszeit 18 h: Umsatz: 78 % (TON = 2848), Aldehydselektivität: >99 %, n/iso = 5.6
Reaktionszeit: 24 h Umsatz: 98 % (TON = 3570), Aldehydselektivität: >99 %, n/iso = 5.6

### Beispiel 23:

Hydroformylierung von 10 ml Vinylcyclooctan mit gleicher Vorgehensweise, wie in Beispiel 10 beschrieben, jedoch mit Ligand aus Beispiel 2 (S-(2-*N,N*-Bis(diphenylphosphinomethyl)aminoethyl)-6^{A}-desoxy-6^{A}-thio-β-cyclodextrin).
Reaktionszeit 18 h: Umsatz: 57 % (TON = 1748), Aldehydselektivität: >99 %, n/iso = 7.1
Reaktionszeit: 70 h Umsatz: 98 % (TON = 3015), Aldehydselektivität: >99 %, n/iso = 7.0

### Beispiel 24:

Hydroformylierung von 10 ml 3,3-Dimethylbuten (Neohexen) mit gleicher Vorgehensweise, wie in Beispiel 10 beschrieben, jedoch mit Ligand aus Beispiel 2 (S-(2-*N,N*-Bis(diphenylphosphinomethyl)aminoethyl)-6^{A}-desoxy-6^{A}-thio-β-cyclodextrin).
Reaktionszeit 18 h: Umsatz: 62 % (TON = 2404), Aldehydselektivität: >99 %, n/iso = 19.6
Reaktionszeit: 70 h Umsatz: 99 % (TON = 3840), Aldehydselektivität: >99 %, n/iso = 20.3

### Beispiel 25:

Hydroformylierung von 10 ml Methylencyclohexan mit gleicher Vorgehensweise, wie in Beispiel 10 beschrieben, jedoch mit Ligand aus Beispiel 2 (S-(2-*N,N*-Bis(diphenylphosphinomethyl)aminoethyl)-6^{A}-desoxy-6^{A}-thio-β-cyclodextrin).
Reaktionszeit 70 h: Umsatz: 45 % (TON = 1872), Aldehydselektivität: >99 %, 100 % *n*-Aldehyd
Reaktionszeit: 150 h Umsatz: 89 % (TON = 3702), Aldehydselektivität: >99 %, 100 % *n*-Aldehyd

### Beispiel 26:

Hydroformylierung von 10 ml 2-Methyl-1-penten mit gleicher Vorgehensweise, wie in Beispiel 10 beschrieben, jedoch mit Ligand aus Beispiel 2 (S-(2-*N,N*-Bis(diphenylphosphinomethyl)aminoethyl)-6^{A}-desoxy-6^{A}-thio-β-cyclodextrin).
Reaktionszeit 70 h: Umsatz: 29 % (TON = 1169), Aldehydselektivität: >99 %, 100 % *n*-Aldehyd
Reaktionszeit: 150 h Umsatz: 47 % (TON = 1895), Aldehydselektivität: >99 %, 100 % *n*-Aldehyd

### Beispiel 27:

Hydroformylierung von 10 ml cis-3-Hexen mit gleicher Vorgehensweise, wie in Beispiel 10 beschrieben, jedoch mit Ligand aus Beispiel 2 (S-(2-*N,N*-Bis(diphenylphosphinomethyl)aminoethyl)-6^{A}-desoxy-6^{A}-thio-β-cyclodextrin).
Reaktionszeit 70 h: Umsatz: 89 % (TON = 2848), Aldehydselektivität: >99 %.
Produkte: 79 % 2-Ethylpentanal, 7 % 2-Methylhexanal, 3 % *n*-Heptanal

### Beispiel 28:

Hydroformylierung von 10 ml trans-3-Hexen mit gleicher Vorgehensweise, wie in Beispiel 10 beschrieben, jedoch mit Ligand aus Beispiel 2 (S-(2-*N,N*-Bis(diphenylphosphinomethyl)aminoethyl)-6^{A}-desoxy-6^{A}-thio-β-cyclodextrin).
Reaktionszeit 70 h: Umsatz: 94 % (TON = 3008), Aldehydselektivität: >99 %.
Produkte: 90 % 2-Ethylpentanal, 4 % 2-Methylhexanal, 0 % *n*-Heptanal

### Beispiel 29:

Hydroformylierung von 10 ml Methyloleat mit gleicher Vorgehensweise, wie in Beispiel 10 beschrieben, jedoch mit Ligand aus Beispiel 2 (S-(2-*N,N*-Bis(diphenylphosphinomethyl)aminoethyl)-6^{A}-desoxy-6^{A}-thio-β-cyclodextrin).
Reaktionszeit 70 h und Reaktionstemperatur 80°C : Umsatz: 21 % (TON = 311)
Reaktionszeit 200 h und Reaktionstemperatur 80°C: Umsatz: 45 % (TON = 666) jeweils mit einer von Aldehydselektivität: >96 %.

### Beispiel 30:

Hydroformylierung von 10 ml Cyclopenten mit gleicher Vorgehensweise, wie in Beispiel 10 beschrieben, jedoch mit Ligand aus Beispiel 2 (S-(2-*N,N*-Bis(diphenylphosphinomethyl)aminoethyl)-6^{A}-desoxy-6^{A}-thio-β-cyclodextrin).
Reaktionszeit 18 h: Umsatz: 67 % (TON = 3806), Aldehydselektivität: >99 %.
Reaktionszeit: 70 h Umsatz: 100 % (TON = 5681), Aldehydselektivität: >99 %.

### Beispiel 31:

Hydroformylierung von 10 ml Cyclohexan mit gleicher Vorgehensweise, wie in Beispiel 10 beschrieben, jedoch mit Ligand aus Beispiel 2 (S-(2-*N,N*-Bis(diphenylphosphinomethyl)aminoethyl)-6^{A}-desoxy-6^{A}-thio-β-cyclodextrin).
Reaktionstemperatur: 80°C.
Reaktionszeit 18 h: Umsatz: 12 % (TON = 480), Aldehydselektivität: >99 %.
Reaktionszeit: 70 h Umsatz: 47 % (TON = 1880), Aldehydselektivität: >99 %.

### Beispiel 32:

Hydroformylierung von 10 ml cis-Cycloocten mit gleicher Vorgehensweise, wie in Beispiel 10 beschrieben, jedoch mit Ligand aus Beispiel 2 (S-(2-*N,N*-Bis(diphenylphosphinomethyl)aminoethyl)-6^{A}-desoxy-6^{A}-thio-β-cyclodextrin).
Reaktionszeit: 70 h Umsatz: 66 % (TON = 2541), Aldehydselektivität: >99 %.

### Beispiel 33:

Hydroformylierung von 10 ml cis-Cyclododecen mit gleicher Vorgehensweise, wie in Beispiel 10 beschrieben, jedoch mit Ligand aus Beispiel 2 (S-(2-*N,N*-Bis(diphenylphosphinomethyl)aminoethyl)-6^{A}-desoxy-6^{A}-thio-β-cyclodextrin).
Reaktionszeit 70 h: Umsatz: 22 % (TON = 556), Aldehydselektivität: >99 %.

### Beispiel 34:

Hydroformylierung von 10 ml 4-Vinylcyclohexen mit gleicher Vorgehensweise, wie in Beispiel 10 beschrieben, jedoch mit Ligand aus Beispiel 2 (S-(2-*N*,*N*-Bis(diphenylphosphinomethyl)aminoethyl)-6^{A}-desoxy-6^{A}-thio-β-cyclodextrin).
Reaktionszeit 70 h: Umsatz: 100 % (TON = 3845), Aldehydselektivität: >99 %, n/iso = 5.6
Es wird selektiv nur die exocyclische Doppelbindung umgesetzt.

### Beispiel 35:

Hydroformylierung von 10 ml 1-Methylcyclopenten mit gleicher Vorgehensweise, wie in Beispiel 10 beschrieben, jedoch mit Ligand aus Beispiel 2 (S-(2-*N,N*-Bis(diphenylphosphinomethyl)aminoethyl)-6^{A}-desoxy-6^{A}-thio-β-cyclodextrin).
Reaktionstemperatur 70°C
Reaktionszeit 70 h: Umsatz: 49 % (TON = 2352), Aldehydselektivität: >99 %.
Reaktionszeit 150 h: Umsatz: 98 % (TON = 4700), Aldehydselektivität: >99 %.
Produkt: 2-Methylcyclopentencarbaldehyd.

### Beispiel 36:

Hydroformylierung von 10 ml Styrol mit gleicher Vorgehensweise, wie in Beispiel 10 beschrieben, jedoch mit Ligand aus Beispiel 2 (S-(2-*N,N*-Bis(diphenylphosphinomethyl)aminoethyl)-6^{A}-desoxy-6^{A}-thio-β-cyclodextrin).
Reaktionszeit 18 h: Umsatz: 73 % (TON = 3175), Aldehydselektivität: >99 %, n/iso = 0.124.

### Beispiel 37:

Hydroformylierung von jeweils 10 ml 1-Octen mit gleicher Vorgehensweise, wie in Beispiel 10 beschrieben, jedoch mit Ligand aus Beispiel 2 (S-(2-*N,N*-Bis(diphenylphosphinomethyl)aminoethyl)-6^{A}-desoxy-6^{A}-thio-β-cyclodextrin). Der Autoklav wird jedoch unter Argon geöffnet und die Katalysatorphase erneut einem Katalyseprozeß zugeführt.

| **Katalyseansatz** | **Umsatz (TON)** | **Reaktionszeit** | **Aldehydselektivität** | **n/iso** |
|---|---|---|---|---|
| neu | 100 % (3172) | 18 h | >99 % | 3.0 |
| 1. Recycling | 93 % (2950) | 18 h | >99 % | 3.0 |
| 2. Recycling | 77 % (2441) | 18 h | >99 % | 2.8 |

### Beispiel 39: Vergleich mit TPPTS

Hydroformylierung von jeweils 10 ml 1-Octen mit gleicher Vorgehensweise, wie in Beispiel 10 beschrieben. Abbruch der Reaktion schon nach acht Stunden. Im Falle von TPPTS wird ein zehnfacher Ligandüberschuß (2.4x10⁻⁴mol) verwendet.

| **Ligand** | **Umsatz (TON)** | **t** | **T** | **n/iso** |
|---|---|---|---|---|
| TPPTS | 0.43 % (14) | 8 h | 120°C | 8.1 |
| Beispiel 2 | 66 % (2092) | 8 h | 60°C | 3.0 |

Das S-(2-*N,N*-Bis(diphenylphosphinomethyl)aminoethyl)-6^{A}-desoxy-6^{A}-thio-β-cycloclextrin-Rhodium-System zeigt eine 153 fach höhere Aktivität in der Hydroformylierung als das TPPTS/Rh-System.

## Patentansprüche

1. β-Cyclodextrin-niodifizierte Diphosphine, dadurch gekennzeichnet, daß eine Bis(Diarylphosphinomethyl)amino- oder eine Bis(Dialkylphosphinomethyl)amino-Einheit direkt oder über einen Spacer an die 6-Position von β-Cyclodextrin oder von einer alkylierten Form von β-Cyclodextrin gebunden ist.

2. Übergangsmetallkomplexe der unter Anspruch 1 definierten Diphosphine, dadurch gekennzeichnet, daß Metallsalze der VIII Gruppe des Periodensystems am Ligand gebunden sind.

3. Verfahren zur Hydroformylierung von 1-Alkenen, 1,1-disubstitutierten Alkenen, inneren disubstitutierten Alkenen, Cycloalkenen und trisubstituierten Alkenen, dadurch gekennzeichnet, daß die genannten Alkene mit CO/H₂ in Gegenwart der unter Anspruch 2 genannten Metallkomplexe als Katalysatoren im Zweiphasensystem umgesetzt werden, wobei die eine Phase Wasser darstellt und den Katalysator enthält, und die zweite Phase entweder das umzusetzende Alken darstellt oder das Alken gelöst in einem organischen Solvens.

4. Verfahren gemäß Anspruch 3, dadurch gekennzeichnet, daß der wäßrigen Phase ein Additiv wie Dimethylformamid zugesetzt wird.

## Claims

1. β-Cyclodextrin modified diphosphines, characterized in that a bis(diarylphosphinomethyl)amino or bis(dialkylphosphinomethyl)amino moiety is bound directly or through a spacer to the 6 position of β-cyclodextrin or an alkylated form of β-cyclodextrin.

2. Transition metal complexes of the diphosphines as defined in claim 1, characterized in that Periodic Table group VIII metal salts are bound to the ligand.

3. A process for the hydroformylation of 1-alkenes, 1,1-disubstituted alkenes, internal disubstituted alkenes, cycloalkenes and trisubstituted alkenes, characterized in that said alkenes are reacted with CO/H₂ in the presence of the metal complexes mentioned in claim 2 as catalysts in a two-phase system, one phase being water which contains the catalyst and the second phase being either the alkene to be reacted or the alkene dissolved in an organic solvent.

4. The process according to claim 3, characterized in that an additive such as dimethylformamide is added to the aqueous phase.

## Revendications

1. Diphosphine modifiée par une β-cyclodextrine, caractérisée en ce qu'une unité bis(diarylphosphinométhyl)amino ou une unité bis(dialkylphosphinométhyl)amino est liée à la position 6 de la β-cyclodextrine ou d'une forme alkylée de β-cyclodextrine, directement ou par l'intermédiaire d'une unité d'espacement.

2. Complexe de métaux de transition de la diphosphine définie à la revendication 1, caractérisé en ce que des sels métalliques du groupe VIII. du système périodique sont liés au ligand.

3. Procédé d'hydroformylation de 1-alcènes, d'alcènes 1,1-disubstitués, d'alcènes disubstitués en interne, de cycloalcènes et d'alcènes trisubstitués, caractérisé en ce que les alcènes nommés sont convertis avec CO/H₂ en présence des complexes métalliques nommés à la revendication 2 en tant que catalyseurs dans un système à deux une phase représentant de l'eau et contenant le catalyseur, la seconde phase représentant soit l'alcène à convertir soit l'alcène dissous dans un solvant organique.

4. Procédé selon la revendication 3, caractérisé en ce que l'on ajoute à la phase aqueuse un additif comme le diméthylformamide.
